# EUROPEAN PATENT APPLICATION

(11) **EP 4 671 361 A1**
(43) Date of publication of application: **31.12.2025**
(21) Application number: 24760305.3
(22) Date of filing: 19.02.2024
(51) Int. Cl.: C12N 5/00

(54) **CELL-DESIGNER MOLECULE IMPARTING PROTEIN ADHESION PROPERTIES TO CELLS**

(30) Priority: 20.02.2023 JP 2023024508
(71) Applicant: The University of Osaka, Osaka 565-0871 (JP)
(72) Inventor: MATSUSAKI, Michiya, Suita-shi, Osaka 565-0871 (JP)
(74) Representative: Hoffmann Eitle
(86) International application number: PCT/JP2024/005767
(87) International publication number: WO 2024/177006

(57) **Abstract**

Provided are: a compound that imparts protein adhesion properties to cells; a method, using the compound, for producing cells having protein adhesion properties; cells adsorbed by the compound; and a cell sheet, a cell aggregation (spheroid, tumor-like body, etc.), an organoid, a three-dimensional tissue, etc. comprising the cells.

## Description

### Technical Field

The present invention relates to the fields of cell engineering, tissue engineering, medical materials, and the like. More particularly, the present invention relates to compounds which impart protein adhesion properties to cells, cells having the compounds adhered thereto, and uses thereof. This application claims priority to Japanese Patent Application No. 2023-24508, the entire contents of which are incorporated herein by reference.

### Background Art

Regenerative medicine aims to regenerate tissues and organs whose function has been weakened or lost due to disease or accident. Regenerative medicine also aims to alleviate the shortage of donors for organ transplants. In regenerative medicine, direct cell transplantation or cell sheet transplantation is carried out. For example, sheets of retinal pigment epithelial cells have been developed for the treatment of age-related macular degeneration (see Patent Literature 1, etc.). Also, for example, myocardial cell sheets have been developed for the treatment of severe cardiomyopathy (see Patent Literature 2, etc.). However, there are some tissues, such as retina, where transplantation of cell sheets is difficult. When cells are transplanted alone, the engraftment rate is low. Even when cell sheets are used, it is often difficult to engraft enough quantity of cells to tissues or organs.

### Citation List

### Patent Literature

Patent Literature 1
   International Publication No. WO2014/030749
Patent Literature 2
   Japanese Patent Laid-Open No. 2011-006490

### Summary of Invention

### Technical Problem

It has been desired to enable the transplantation of cells into tissues and organs where direct cell transplantation or cell sheet transplantation is difficult, and to establish enough quantity of cells in the tissue or organ at the time of transplantation.

### Solution to Problem

The present inventors have conducted diligent study to solve the above problems and discovered that protein adhesion properties can be imparted to cells by adsorbing a compound comprising a hydrophilic polymer or a derivative thereof, a bile acid or a derivative thereof, and a protein-directed substance to the cells, thereby completing the present invention. Specifically, the present invention provides the followings:
(1) A compound comprising a hydrophilic polymer or a derivative thereof, a bile acid or a derivative thereof, and a protein-directed substance;
(2) The compound according to (1), wherein the hydrophilic polymer is selected from the group consisting of polyethylene glycol, polyvinyl alcohol, polyglutamic acid, polyaspartic acid, polylysine and polyhistidine.
(3) The compound according to (1) or (2), wherein the hydrophilic polymer is polyethylene glycol or polyvinyl alcohol.
(4) The compound according to any one of (1) to (3), wherein the bile acid is selected from the group consisting of cholic acid, chenodeoxycholic acid, deoxycholic acid, lithocholic acid, ursodeoxycholic acid, hyodeoxycholic acid, glycocholic acid and taurocholic acid.
(5) The compound according to any one of (1) to (4), wherein the bile acid is deoxycholic acid or cholic acid.
(6) The compound according to any one of (1) to (5), wherein the protein-directed substance is an extracellular matrix-directed substance.
(7) The compound according to any one of (1) to (6), wherein the protein-directed substance is a collagen-directed substance.
(8) A method for producing cells having protein adhesion properties, comprising adsorbing the compound according to any one of (1) to (7) to the cells.
(9) An agent for imparting protein adhesion properties to cells, comprising the compound according to any one of (1) to (7).
(10) A kit for imparting protein adhesion properties to cells, comprising the compound according to any one of (1) to (7).
(11) A Cell adsorbed with the compound according to any one of (1) to (7).
(12) A graft containing the cells according to (11).
(13) The graft according to (12), which is in the form of cell mass, organoid, three-dimensional tissue, or paste.

### Advantageous Effects of Invention

The present invention provides a compound which imparts protein adhesion properties to cells, cells to which the compound is adsorbed, a cell sheet containing the cells, and the like. Cells to which the compound of the present invention is adsorbed exhibit similar proliferation properties to cells to which the compound is not adsorbed. Even after re-thawing after cryopreservation, cells to which the compound of the present invention is adsorbed maintain a high viability rate.

### Brief Description of Drawings

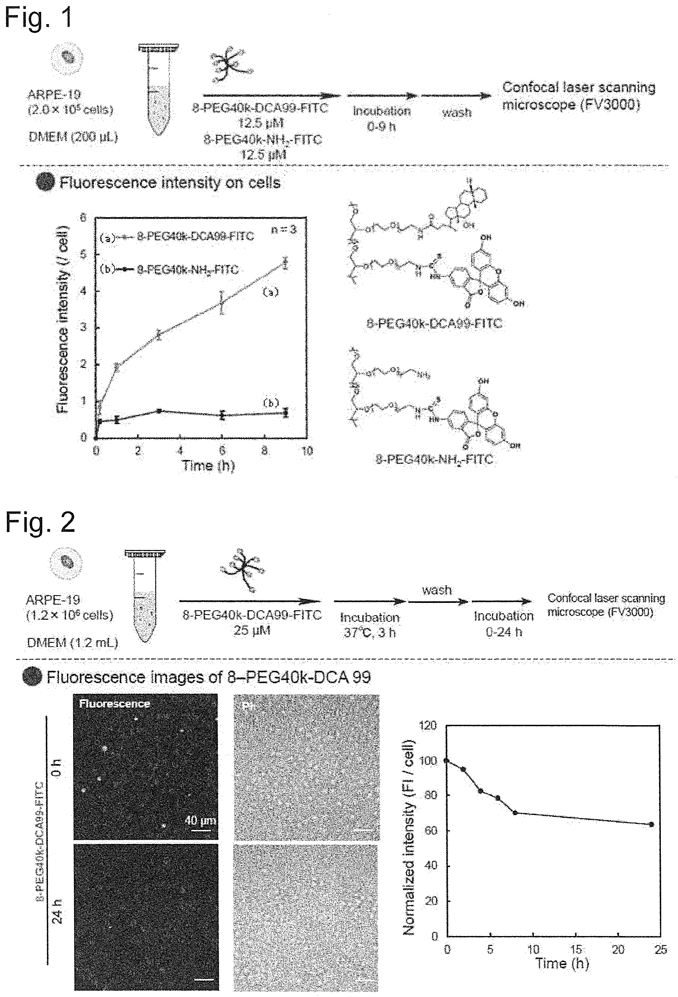

[Figure 1] The upper part is a scheme showing the experimental procedure of Example 1. The lower left panel shows the results of incubating ARPE with 8-PEG40k-DCA99-FITC and measuring the fluorescence intensity on cells over time. The lower right panel shows the structures of 8-PEG40k-DCA99-FITC and 8-PEG40k-NH₂-FITC.
[Figure 2] The upper part is a scheme showing the experimental procedure of Example 2. The lower left panels show confocal laser scanning microscopic images and phase-contrast microscopic images of the cells before and after 24 hours of incubation. The lower right panel shows the results of measuring the fluorescence over time of the cells obtained by incubating ARPE with 8-PEG40k-DCA99-FITC at 37°C for 3 hours.

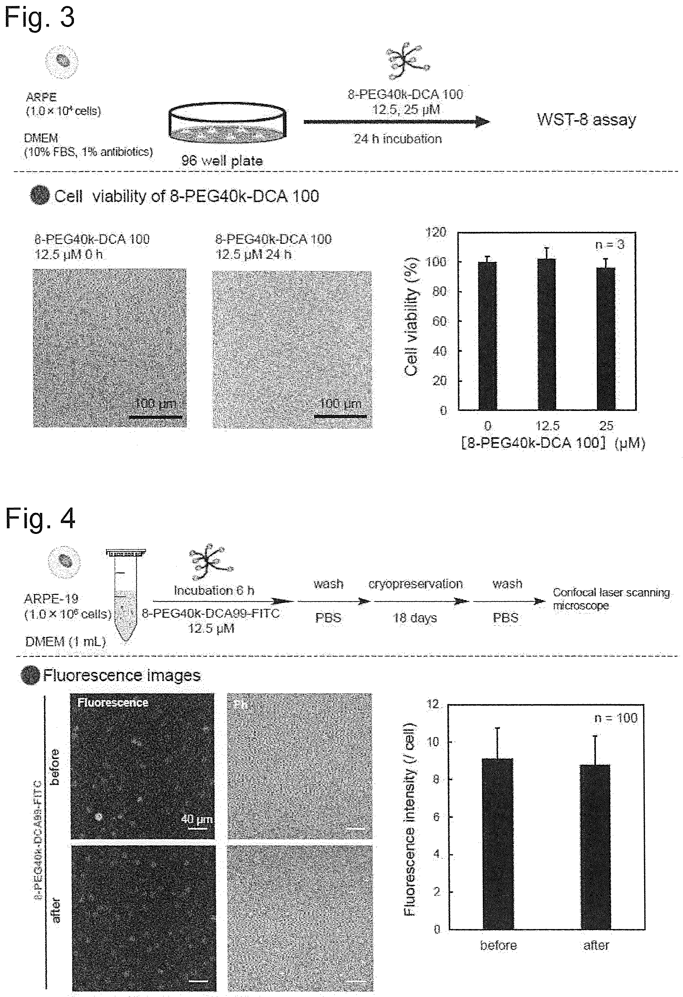

[Figure 3] The upper part is a scheme showing the experimental procedure of Example 3. In the lower left part, the left panel shows a phase-contrast microscopic image of the cells before 24 hours of incubation, and the right panel shows a phase-contrast microscopic image of the cells after 24 hours of incubation. The lower right panel shows the viability rate determined by incubating cells with 8-PEG40k-DCA100 (0 µM, 12.5 µM, 25 µM) for 24 hours and counting the number of viable cells using WST-8.
[Figure 4] The upper part is a scheme showing the experimental procedure of Example 4. The lower left panels show confocal laser scanning microscopic images and phase-contrast microscopic images of the cells before and after freezing. The lower right panel shows the results of investigating the stability of 8-PEG40k-DCA99-FITC adsorbed to cells after cryopreservation (18 days) by measuring the fluorescence intensity.

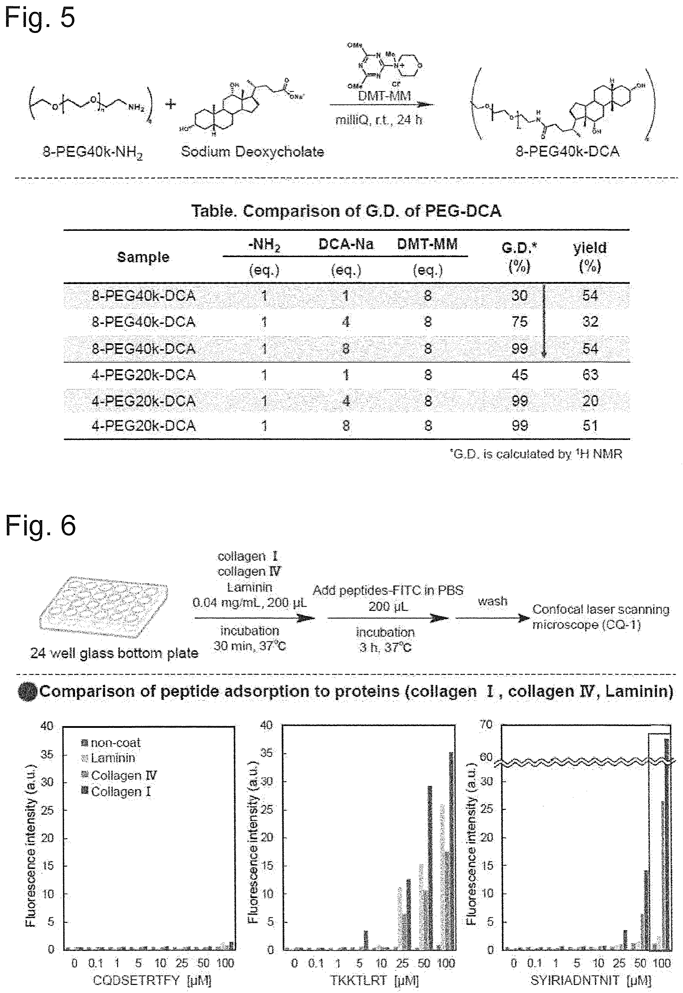

[Figure 5] The upper part is a scheme showing the synthesis procedure of 8-PEG40k-DCA. The lower part is a table showing the introduction rate of deoxycholic acid (DCA) into PEG when PEG-DCA was synthesized by changing the ratio of PEG, DCA, and DMT-MM.
[Figure 6] The upper part is a scheme showing the experimental procedure of Example 6. The lower panels show the results of investigating the bonding ability of three FITC-labeled collagen-binding peptides (CQDSETRTFY, TKKTLRT, SYIRIADNTNIT) to type I collagen, type IV collagen, and laminin by measuring the fluorescence intensity.

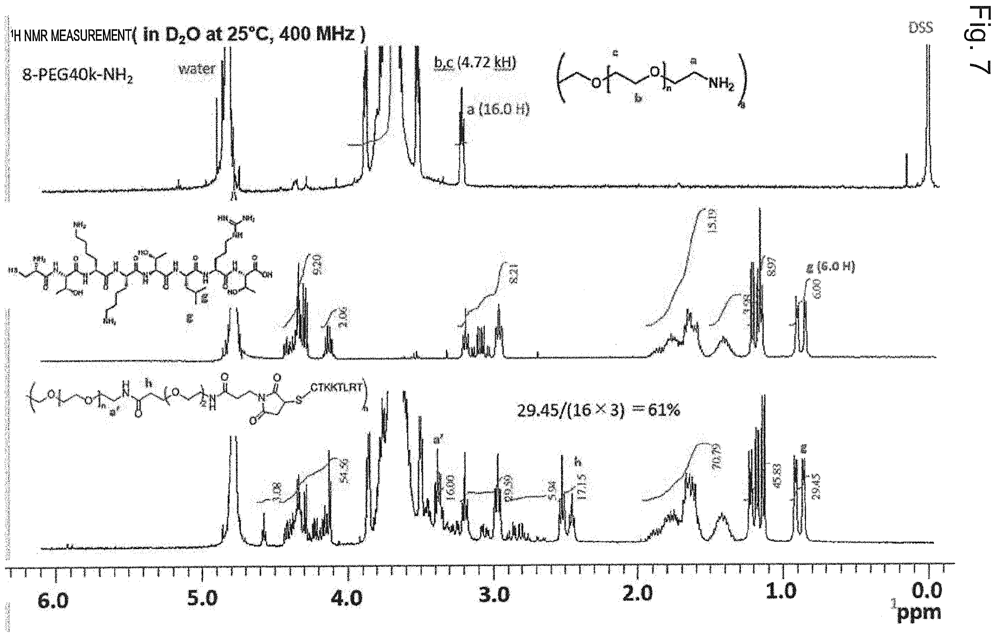

[Figure 7] The upper part is a ¹H NMR chart of 8-PEG40k-NH₂ (8-branched PEG with molecular weight of 40,000 having an amino group at the terminal end). The middle part is a ¹H NMR chart of the collagen-binding peptide CTKKTLRT. The lower part is a ¹H NMR chart of 8-PEG40k-NH₂ to which the collagen-binding peptide CTKKTLRT is bonded via maleimide. The symbols a, b, etc., in the charts and structural formulas are symbols for proton assignment.

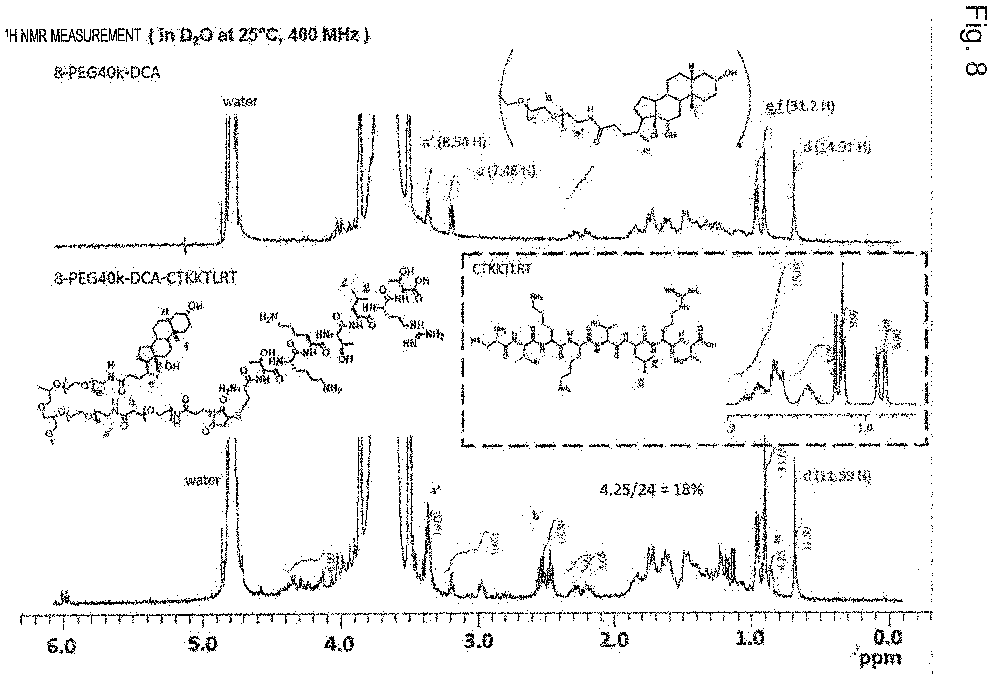

[Figure 8] The upper part is a ¹H NMR chart of 8-PEG40k-DCA (8-branched PEG with molecular weight of 40,000 introduced with deoxycholic acid). The lower part is a ¹H NMR chart of 8-PEG40k-DCA-CTKKTLRT (8-branched PEG with molecular weight of 40,000 introduced with deoxycholic acid and collagen-binding peptide CTKKTLRT). The symbols a', f, etc., in the charts and structural formulas are symbols for proton assignment.

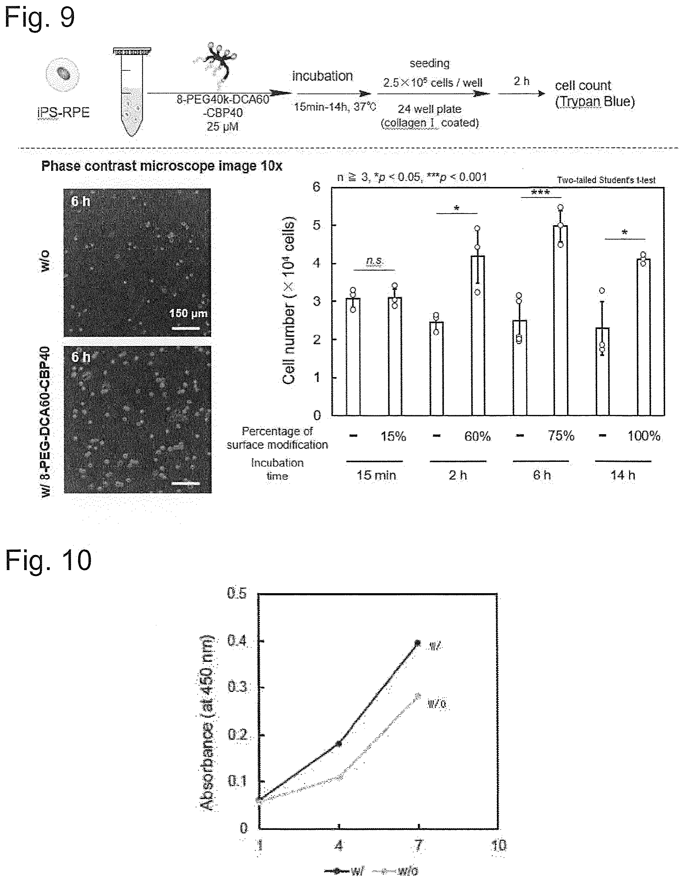

[Figure 9] The upper part is a scheme showing the experimental procedure of Example 8. In the lower part, the upper photograph is a phase-contrast microscopic image of iPS-RPE cells and the lower photograph is a phase-contrast microscopic image of iPS-PRE cells coated with 8-PEG40k-DCA60-CBP40 seeded on the surface of a collagen-coated dish and washed after 2 hours. The graph in the lower part shows the results of investigating the effect of incubation time of 8-PEG40k-DCA60-CBP40 with iPS-RPE on collagen adhesion of iPS-RPE.
[Figure 10] This graph shows the results of comparing the proliferation of iPS-RPE to which 8-PEG40k-DCA60-CBP40 is adsorbed (w/) and the proliferation of iPS-RPE to which 8-PEG40k-DCA60-CBP40 is not adsorbed (w/o).

### Description of Embodiments

In one aspect, the present invention provides a compound comprising a hydrophilic polymer or a derivative thereof, a bile acid or a derivative thereof, and a protein-directed substance. As used herein, the above compound can be referred to as "compound of the present invention" or "cell-designer molecule of the present invention." The compound of the present invention can impart protein adhesion properties to cells.

A hydrophilic polymer is a polymer which contains charged or polar functional groups and is capable of dissolving in and interacting with water and other polar substances. The hydrophilic polymer contained in the compound of the present invention carries a bile acid or a derivative thereof, and a protein-directed substance or a derivative thereof. The hydrophilic polymer contained in the compound of the present invention can be of any type. The molecular weight of the hydrophilic polymer is not particularly limited, and can be, for example, several thousand to several hundred thousand. Nor the structure of the hydrophilic polymer is particularly limited, and can be linear, branched, cyclic, or the like. A preferred hydrophilic polymer is one which has low or no cytotoxicity, or one which has high biocompatibility.

Examples of the hydrophilic polymer include polyethylene glycol, polyvinyl alcohol, poly(vinylpyrrolidone), polyvinylamide, polyacrylic acid, polyacrylamide, polyamino acids, polysaccharides, and proteins. Examples of the preferred hydrophilic polymer include polyethylene glycol (PEG), polyvinyl alcohol, polyglutamic acid, polyaspartic acid, polylysine, and polyhistidine. More preferred examples of the hydrophilic polymer include PEG and polyvinyl alcohol. An even more preferred example of the hydrophilic polymer is PEG. It goes without saying that the hydrophilic polymer contained in the compound of the present invention is not limited to the above examples.

When the hydrophilic polymer is PEG, it can be any type of PEG. The form of PEG is not particularly limited, and linear, Y-shaped, branched, etc., can be used. Preferably, branched PEGs are used in the compound of the present invention. The number of branches can be 2, 3, 4, 5, 6, 7, 8, 9, 10, 11, 12 or more, for example, 2 to 20 branches, 2 to 10 branches, 2 to 8 branches, 3 to 12 branches, 3 to 10 branches, 3 to 9 branches, 4 to 12 branches, 4 to 10 branches, 4 to 8 branches, etc. Highly branched PEGs are preferred. Four-arm and eight-arm PEGs are commercially available and can be preferably used. The molecular weight of PEG is not particularly limited, and PEG can have molecular weight of several thousand to tens of thousands or hundreds of thousands, for example, about 10,000 to about 200,000, about 10,000 to about 100,000, about 20,000 to about 100,000, or about 40,000 to about 80,000.

Derivatives of the hydrophilic polymer can be appropriately produced by those skilled in the art, and are also commercially available. The functional groups in the hydrophilic polymer can be amidated, esterified, alkylated, halogenated, or the like by known methods. For example, an amino group, a maleimide group, a carboxyl group, or the like can be bonded to the terminal end of polyethylene glycol by known methods. Derivatives of the hydrophilic polymer can be selected depending on the bile acid or its derivative to be bonded to the hydrophilic polymer, and on the type and structure of the protein-directed substances.

Bile acid is steroid compound which is biosynthesized from cholesterol in the mammalian liver. Bile acid contributes to the adsorption of the compound of the present invention to cells. A variety of bile acids is known, and an appropriate one can be selected and used in the compound of the present invention. Bile acids include primary bile acids such as cholic acid and chenodeoxycholic acid; secondary bile acids such as deoxycholic acid, lithocholic acid, ursodeoxycholic acid, and hyodeoxycholic acid; and conjugated bile acids such as glycocholic acid and taurocholic acid; any of which can be used in the compound of the present invention. Preferably, deoxycholic acid or cholic acid is used. More preferably, deoxycholic acid (DCA) is used.

Derivatives of bile acids are known, can be appropriately synthesized, and are also commercially available. For example, any hydroxyl group of the bile acid can be attached or detached, or can be alkylated, or the like. Also, for example, the carboxyl groups of bile acids can be esterified. The derivatives of bile acids are not limited to those mentioned above.

A protein-directed substance is a substance which has a high affinity or specific bonding ability to a protein. A protein directed by a protein-directed substance can be, for example, one present on the cell surface or one present in the extracellular matrix. A protein-directed substance contributes to the ability of the compound of the present invention to impart protein adhesion properties to cells. An example of protein-directed substance is an extracellular matrix-directed substance. In general, the extracellular matrix includes collagen, laminin, fibronectin, elastin, etc., and therefore the extracellular matrix-directed substance is preferably a substance which has high affinity or specific bonding ability to these proteins. When collagen is abundant in the extracellular matrix, the extracellular matrix-directed substance can be a collagen-directed substance. The protein-directed substance can also be a substance which has a high affinity or specific bonding ability to polysaccharides present on the cell surface or polysaccharides contained in the extracellular matrix, in addition to proteins. The protein-directed substance can be of any type, such as a peptide, a sugar, a lipid, or a nucleic acid. Protein-directed substances can be naturally occurring or artificially derived. Those skilled in the art can synthesize protein-directed substances using known methods and materials. The protein-directed substance can be a commercially available compound.

Examples of the protein-directed substances include, but are not limited to, CD42b which recognizes the vWF factor expressed in damaged blood vessels; tannic acid which adsorbs to the surface of myocardium; DOPA which adsorbs to the surface of intestinal epithelial cells; and serine which adsorbs to the surface of renal epithelial cells. Examples of the extracellular matrix-directed substances include, but are not limited to, a collagen-directed substance which recognizes collagen; and heparin which recognizes fibronectin. Examples of collagen-directed substances include, but are not limited to, collagen-binding peptides such as CQDSETRTFY, TKKTLRT, and SYIRIADNTNIT.

When cells adsorbing the compound of the present invention, or a cell sheet or a graft containing cells adsorbing the compound of the present invention, are transplanted into a tissue or organ, the hydrophilic polymer or derivative thereof, the bile acid or derivative thereof, and the protein-directed substance can be appropriately selected depending on the type of cells used for transplantation, the composition of the extracellular matrix contained in the recipient tissue or organ, the transplant site, the form and physical properties of the graft, etc. The engraftment rate of cells adsorbing the compound of the present invention, or that of a cell sheet or a graft containing cells adsorbing the compound of the present invention can be improved by selecting a protein-directed substance, taking into consideration the composition of the extracellular matrix contained in the recipient tissue or organ.

The compound of the present invention can be synthesized by bonding the hydrophilic polymer or derivative thereof, the bile acid or derivative thereof, and the protein-directed substance. The compound of the present invention can be synthesized using any known method. Typically, the compound of the present invention is synthesized by covalently bonding the bile acid or derivative thereof, and the protein-directed substance to the hydrophilic polymer or derivative thereof. Functional groups in the hydrophilic polymer or derivative thereof, functional groups in the bile acid or derivative thereof, and functional groups in the protein-directed substance can be used for bonding. The bile acid or derivative thereof, and the protein-directed substance can be bonded to any position of the hydrophilic polymer or derivative thereof, but is preferably bonded near or at the terminal end. These bonding modes can be appropriately selected by those skilled in the art. For example, the bile acid or derivative thereof, and the protein-directed substance can be bonded to the hydrophilic polymer by known techniques such as amide formation, ester formation, nucleophilic substitution reaction (e.g., S_{N}2 reaction using a tosyl group), and click reaction. For example, PEG having an amino group at the terminal end can be used, and the amino group can be condensed with a carboxyl group of deoxycholic acid using a condensing agent such as DMT-MM to form an amide (peptide bond), thereby bonding PEG to deoxycholic acid. In addition, for example, PEG having an amino group or carboxyl group at the terminal end and a peptidic protein-directed substance can be used to form a peptide bond in the same manner as described above, thereby bonding the PEG to the peptidic protein-directed substance. Alternatively, for example, PEG having a carboxyl group at the terminal end can be used, and an ester bond can be formed between the carboxyl group and the hydroxyl group of the protein-directed substance, thereby bonding the PEG to the protein-directed substance. The bondings of hydrophilic polymer or derivative thereof, the bile acid or derivative thereof, and the protein-directed substance are not limited to the above examples. PEG having a functional group attached to the terminal end can be synthesized using known methods and materials. PEG having a functional group attached to the terminal end is commercially available, and such PEG can also be used. The ratio of the hydrophilic polymer or its derivative, the bile acid or its derivative, and the protein-directed substance can also be appropriately determined.

The hydrophilic polymer contained in the compound of the present invention can be one type, or two or more types. The bile acid contained in the compound of the present invention can be one type, or two or more types. The protein-directed substance contained in the compound of the present invention can be one type, or two or more types. The compound of the present invention may contain a molecule which promotes tissue-specific bonding, a therapeutic agent, a functional molecule such as a label, or the like.

In another aspect, the present invention provides a method for producing cells having protein adhesion properties, which comprises adsorbing the compound of the present invention to cells.

The cells adsorbing the compound of the present invention can be of any type and origin, and can be appropriately selected depending on the purpose. The purpose is, for example, regenerative medicine, in particular transplantation. The cells can be cardiomyocytes, retinal pigment epithelial cells, hepatocytes, vascular endothelial cells, mesenchymal stem cells, osteoblasts, chondrocytes, myoblasts, or the like. The cells may be cells isolated directly from an organism or may be an established cell line. The cells can be derived from stem cells, such as iPS cells or ES cells. The cells absorbing the compound of the present invention are not limited to those exemplified above.

In the above production method, protein adhesion properties can be imparted to cells which do not have protein adhesion properties, or the protein adhesion properties of cells which have protein adhesion properties can be enhanced.

The compound of the present invention can be adsorbed to cells by contacting the compound of the present invention with cells. For example, the compound of the present invention can be adsorbed to cells by incubating the compound and cells in a medium. Preferably, the medium is an aqueous medium, which can be a cell culture medium. Media for cell culture are known and can be appropriately selected depending on the type of cells. Conditions such as temperature and time for incubation can also be appropriately determined by those skilled in the art. The incubation temperature can be between 280°C and 38°C, such as between 30°C and 37°C. The incubation time can be from several tens of minutes to several tens of hours, for example, from 15 minutes to 36 hours, or from 2 hours to 24 hours. For example, the cells can be incubated in DMEM medium at 37°C for 12 to 36 hours. The ratio of the compound of the present invention to cells (the concentration of the compound of the present invention in the medium) may also be appropriately determined by those skilled in the art. For example, the compound of the present invention may be incubated at a concentration of several µM to several tens of µM with respect to 10⁴ to 10⁸ cells/mL.

In a further aspect, the present invention provides an agent containing the compound of the present invention for imparting protein adhesion properties to cells. The dosage form of the above agent can be solid (powder, granules, etc.), liquid (solution, suspension, etc.), or semi-solid (paste, etc.). The above description regarding the method for producing cells having protein adhesion properties can be applied to the usage and dosage of the above agent.

In a further aspect, the present invention provides a kit containing the compound of the present invention for imparting protein adhesion properties to cells. The above kit contains the compound of the present invention as a component. For example, the above agent can be included in the package of the above kit. Typically, the kit will come with an instruction manual.

In a further aspect, the present invention provides cells adsorbing the compound of the present invention. The compound of the present invention can be adsorbed to cells to improve the directionality and selectivity to a target organ or tissue. Cells adsorbing the compound of the present invention have a viability rate similar to that of cells without adsorption of the compound of the present invention. Therefore, when cells adsorbing the compound of the present invention are used for transplantation, there is a possibility that the success rate of transplantation will be increased.

When the cells adsorbing the compound of the present invention are administered to a living body, any administration route which is used for administering cells to a living body can be used. Examples of administration route include, but are not limited to, injection into the affected area, injection into a vein, and catheter administration.

In a further aspect, the present invention provides a cell sheet containing cells adsorbing the compound of the present invention. In a further aspect, the present invention provides a graft containing cells adsorbing the compound of the present invention. The cells adsorbing the compound of the present invention can themselves be used as a graft, and a graft containing the above cells can also be prepared and used for transplantation. Examples of the form of the graft include, but are not limited to, a cell sheet, a cell mass (spheroid, embryoid body, etc.), an organoid, a three-dimensional tissue, and a paste. Grafts of various shapes can be prepared using known methods. When such cell sheets or grafts are used for transplantation, they have high adhesion to organs or tissues, and the viability rate of the cells in the sheets or grafts is also high. Therefore, the graft is suitable for regenerative medicine, particularly for transplantation. Examples of organ and tissue into which the graft of the present invention can be transplanted include, but are not limited to, heart, retina, damaged blood vessels, intestine, kidney, bone, and tumor.

Hereinafter, the present invention is described in more detail and specifically with reference to examples, but the scope of the present invention is not limited to these examples.

### Example 1

The bonding properties of 8-PEG40k-DCA99-FITC (compound in which deoxycholic acid (DCA) and FITC are introduced at a ratio of 99:1 to 8-branched PEG with molecular weight of 40,000 - see the lower part of Figure 1) to cells were investigated. The cells used were from retinal pigment epithelial cell line (ARPE) (Examples 1 to 5). As shown in the upper part of Figure 1, ARPE and 8-PEG40k-DCA99-FITC were incubated, and the fluorescence intensity on cells was measured over time. As a control system, 8-PEG40k-HN₂-FITC (8-PEG40k having molecular weight of 40,000 introduced with a few percent of FITC alone - see the lower part of Figure 1) was used. As shown in the lower graph of Figure 1, it was found that the fluorescence intensity increased over time, and the amount of 8-PEG40k-DCA99-FITC adsorbed to the ARPE surface increased.

### Example 2

The stability of 8-PEG40k-DCA99-FITC adsorbed to cells was investigated. As shown in the upper part of Figure 2, ARPE and 8-PEG40k-DCA99-FITC were incubated at 37°C for 3 hours, and the fluorescence of the cells obtained was measured over time. As shown in the lower photographs of Figure 2, there was no significant change in the fluorescence of the cells even after 24 hours of incubation, and the decrease in fluorescence intensity was about 30% after 24 hours of incubation. These results demonstrated that 8-PEG40k-DCA99-FITC adsorbed to cells was not easily removed.

### Example 3

The viability rate of the cells adsorbing 8-PEG40k-DCA100 (8-branched PEG with molecular weight of 40,000 introduced with DCA alone) was investigated. As shown in the upper part of Figure 3, cells were incubated with 8-PEG40k-DCA100 (0 µM, 12.5 µM, 25 µM) for 24 hours, and the viability rate was determined by counting the number of viable cells using WST-8. As shown in the lower part of Figure 3, it was found that adsorption of 8-PEG40k-DCA100 did not affect the viability rate of cells.

### Example 4

The stability of 8-PEG40k-DCA99-FITC adsorbed to cells after cryopreservation was investigated. As shown in the upper part of Figure 4, after incubating the cells with 8-PEG40k-DCA99-FITC for 6 hours, the cells were cryopreserved for 18 hours. The frozen and thawed cells were washed with PBS, and the fluorescence intensity was measured using a confocal laser scanning microscope. As shown in the photographs and graph in the lower part of Figure 4, no change was observed in the fluorescence intensity on cells before and after cryopreservation. These results demonstrated that 8-PEG40k-DCA99-FITC was stably adsorbed to the cells even after the cells were freeze-dried. Furthermore, the number of viable cells was counted before and after freezing using trypan blue staining. The results are shown in Table 1. It was found that the cells adsorbing 8-PEG40k-DCA99-FITC maintained a high viability rate even after cryopreservation and thawing (viability rate before cryopreservation: 93%, viability rate after cryopreservation: 90%).

**[Table 1]**

| | **Total concentration (/mL)** | **LIVE (/mL)** | **DEAD (/mL)** |
|---|---|---|---|
| Before cryopreservation | 8.21 × 10⁵ | 7.71 × 10⁵ (93%) | 4.98 × 10⁴ (6%) |
| After cryopreservation | 5.86 × 10⁵ | 5.28 × 10⁵ (90%) | 5.86× 10⁴ (10%) |

| | | | |
|---|---|---|---|
| Trypan blue staining | | | |

From these results, it is considered that cells and sheets thereof adsorbing the compound of the present invention have high engraftment rate in tissues and organs and high viability rate, and can be engrafted and function for a long period of time.

### Example 5

The effect of the number of polymer branches on cell adhesion ability was investigated. Cells (1 x 10⁶ cells/mL) and 8-PEG40k-DCA99-FITC (8-branched PEG) (25 µM) or FITC-PEG5k-DCA95 (unbranched) (25 µM) were incubated in DMEM/F12 at 37°C for 6 hours, washed, and the fluorescence intensity was observed using a confocal laser scanning microscope. The fluorescence intensity per cell adsorbing 8-branched PEG was about 2.1, and the fluorescence intensity per cell adsorbing unbranched PEG was adsorbed was about 1.4. These results demonstrated that the compound bonded with branched PEG-DCA was more adsorbed to cells.

### Example 6

The selectivity of collagen-binding peptides for components of the extracellular matrix was investigated. As shown in the upper part of Figure 6, three types of FITC-labeled collagen-binding peptides (CQDSETRTFY, TKKTLRT, SYIRIADNTNIT) were added to wells coated with type I collagen, type IV collagen, or laminin, and incubated at 37°C for 3 hours. After washing, the fluorescence intensity was measured using a confocal laser scanning microscope. As shown in the lower part of Figure 6, TKKTLRT and SYIRIADNTNIT bound to type I collagen, type IV collagen, and laminin in a concentration-dependent manner. The fluorescence intensity of SYIRIADNTNIT was stronger than that of TKKTLRT (100 µM, box). The selectivity of TKKTLRT was highest for type I collagen, and decreased in the order of type I collagen > laminin > type IV collagen. The selectivity of SYIRIADNTNIT was highest for type I collagen, and decreased in the order of type I collagen > type IV collagen > laminin.

### Example 7

### (1) Preparation of 8-PEG40k-DCA

8-PEG40k-DCA was prepared according to the procedure shown in the upper part of Figure 5. 8-PEG40k-NH₂ (8-branched PEG with molecular weight of 40,000 having an amino group at the terminal end) (purchased from Sigma-Aldrich) was condensed with deoxycholic acid sodium salt using DMT-MM (4-(4,6-dimethoxy-1,3,5-triazin-2-yl)-4-methylmorpholinium chloride) to form an amide bond, thereby obtaining 8-PEG40k-DCA. 4-PEG20k-NH₂ (4-branched PEG with molecular weight of 20,000 having an amino group at the terminal end) (purchased from Sigma-Aldrich) was used to obtain 4-PEG20k-DCA in the same manner as above. The ratios of the amino group of PEG, sodium deoxycholic acid, and DMT-MM were also investigated. As shown in the lower table of Figure 5, the introduction rate increased with an increase in the ratio of sodium deoxycholic acid (up to 99%).

### (2) Preparation of 8-PEG40k-DCA99-FITC

Eight equivalents of DCA and DMT-MM were added to 8-PEG40k-NH₂, and the mixture was reacted by stirring in water at room temperature for 24 hours. After dialysis against ethanol and water, the product was recovered by freeze-drying. The yield was 54%.

### (3) Preparation of the compound of the present invention introduced with collagen-directed peptide

Maleimide-PEG-NHS (TCI) and 8-PEG-DCA45-NH₂ were stirred in PBS for 90 minutes, and the collagen-directed peptide CTKKTLRT was added and reacted at room temperature for 24 hours. The product 8-PEG40k-DCA-CTKKTLRT (8-branched PEG with molecular weight of 40,000 introduced with deoxycholic acid and collagen-binding peptide CTKKTLRT (referred to as 8-PEG40k-DCA60-CBP40) was recovered by dialysis and lyophilization. The product was confirmed by ¹H NMR (Figures 7 and 8). Here, DCA60 means that the introduction rate of DCA into PEG is 60%, and CBP40 means that the introduction rate of CTKKTLRT into PEG is 40%.

### Example 8

The adhesion of cells adsorbing the compound of the present invention to proteins was investigated. Specifically, investigation was made whether the adsorption of 8-PEG40k-DCA60-CBP40 to iPS cell-derived retinal pigment epithelial cells (abbreviated as iPS-RPE) enhances the collagen adhesion ability of iPS-RPE.
(1) Experimental method
(i) Preparation of collagen-coated wells
1. 500 µL of human collagen I diluted with 1N HCl was added to a 24-well plate to give 3 µg/cm².
2. After coating at room temperature for 1 hour, 500 µL of 1×DPBS was added and washed twice in total.

(ii) Adhesion Experiment
1. iPS-RPE cells were added to 1.5 mL Eppendorf tubes to give 1 x 10⁶ cells, and then the supernatant medium was removed by centrifugation. (4 tubes were prepared.)
2. iPS-RPE medium (25 µM) containing 8-PEG40k-DCA60-CBP40 was added to the cell suspensions to give 1 x 10⁶ cells/mL, and the cell suspensions were incubated at 37°C for 15 minutes, 2 hours, 6 hours, and 14 hours, respectively.
3. After incubation, the cell suspensions were centrifuged at 300 ×*g* for 5 minutes, the solution was removed, and 1 mL of iPS-RPE medium was added again and washed.
4. The cells suspended in fresh iPS-RPE medium were seeded in collagen-coated wells at 2.5×10⁵ cells/well, and incubated at 37°C for 2 hours.
5. After 2 hours, 500 µL of 1×DPBS was added, and non-adherent cells were washed three times in total.
6. TryplLE (10X) (400 µL) was added, and the mixture was incubated at 37°C for 20 minutes to detach the cells.
7. After detachment, medium was added and the cells were collected in 1.5 mL Eppendorf tubes, centrifuged at 300 ×*g* for 5 minutes, the supernatant was removed, and the cells were suspended again in 100 µL of iPS-RPE medium.
8. The number of cells was counted using trypan blue, and the volume of the cell suspension was also measured.
9. The number of adherent cells was calculated using the results from step 8.
The composition of the iPS-RPE medium was as follows:

| | |
|---|---|
| MEMα (Thermo Fisher, 12571-063) | 91.3% |
| KSR (Thermo Fisher, 10828-028) | 5% |
| N2 supplement (Thermo Fisher, 17502-048) | 1% |
| Hydrocortisone (Sigma, H0888-1G) | 55 nM |
| Taurine, 10G (Sigma, T0625-10G) | 250 µg/ml |
| Triiodothyronine (T3) (Sigma, T5516-1MG) | 14 pg/ml |
| Gentamicin (Thermo Fisher, 15750-060) | 25 µg/ml |

(2) Experimental results

The results are shown in Figure 9. The adsorption rate of 8-PEG40k-DCA60-CBP40 to iPS-RPE (Percentage of surface modification in Figure 8) increased with an increase in incubation time. The number of iPS-RPE which adhered to collagen, the iPS-RPE adsorbing 8-PEG40k-DCA60-CBP40 obtained by incubation for 2 hours or more, was approximately twice the number of the cells which adhered to collagen, the cells without adsorption of 8-PEG40k-DCA60-CBP40. These results show that the collagen adhesion ability of cells was enhanced by adsorption of 8-PEG40k-DCA60-CBP40.

### Example 9

The proliferation of cells adsorbing the compound of the present invention was compared with that of cells without adsorption of the compound of the present invention. Specifically, the proliferation of iPS-RPE adsorbing 8-PEG40k-DCA60-CBP40 was compared to that of iPS-RPE without adsorption of 8-PEG40k-DCA60-CBP40.
(1) Experimental method
   (i) Preparation of collagen-coated wells
      1. 200 µL of human collagen I diluted with 1N HCl was added to a 24-well plate to give 3 µg/cm².
      2. After coating at room temperature for 1 hour, 200 µL of 1×DPBS was added and washed twice in total.
   (ii) Proliferation Experiment
      1. iPS-RPE cells were added to a 1.5 mL Eppendorf tube to give 1 x 10⁶ cells, and then the supernatant medium was removed by centrifugation.
      2. iPS-RPE medium (25 µM) containing 8-PEG40k-DCA60-CBP40 was added to the cell suspensions to give 1 x 10⁶ cells/mL, and the cell suspensions were incubated at 37°C for 6 hours.
      3. After incubation, the cell suspensions were centrifuged at 300 ×*g* for 5 minutes, the solution was removed, and 1 mL of iPS-RPE medium was added again and washed.
      4. The cells suspended in fresh iPS-RPE medium were seeded in collagen-coated wells at 1.25×10⁵ cells/well, and incubated at 37°C for 2 hours.
      5. After 2 hours, 200 µL of 1×DPBS was added, and non-adherent cells were washed three times in total. The cells were cultured as is, and the proliferation ability of the cells was investigated by WST-8 assay after 1, 4, and 7 days.
   (iii) Cell count (performed after 1, 4, and 7 days)
      1. The wells prepared in step (ii) 5 were washed with 200 µL of 1×DPBS.
      2. 200 µL of iPS-RPE medium (10%, WST-8 reagent) was added, and incubated at 37°C for 40 minutes.
      3. 100 µL of the supernatant was taken and transferred to a 96-well plate.
      4. The 96-well plate containing the supernatant was placed in a plate reader and the absorbance at 450 nm was measured.
(2) Experimental results

The results are shown in Figure 10. The cells adsorbing 8-PEG40k-DCA60-CBP40 tended to proliferate better than the cells without adsorption of 8-PEG40k-DCA60-CBP40.

### Industrial Applicability

The present invention can be used in the fields of cell engineering, tissue engineering, medical materials, medicine, and the like.

## Claims

1. A compound comprising a hydrophilic polymer or a derivative thereof, a bile acid or a derivative thereof, and a protein-directed substance.

2. The compound according to claim 1, wherein the hydrophilic polymer is selected from the group consisting of polyethylene glycol, polyvinyl alcohol, polyglutamic acid, polyaspartic acid, polylysine and polyhistidine.

3. The compound according to claim 1, wherein the hydrophilic polymer is polyethylene glycol or polyvinyl alcohol.

4. The compound according to claim 1, wherein the bile acid is selected from the group consisting of cholic acid, chenodeoxycholic acid, deoxycholic acid, lithocholic acid, ursodeoxycholic acid, hyodeoxycholic acid, glycocholic acid and taurocholic acid.

5. The compound according to claim 1, wherein the bile acid is deoxycholic acid or cholic acid.

6. The compound according to claim 1, wherein the protein-directed substance is an extracellular matrix-directed substance.

7. The compound according to claim 1, wherein the protein-directed substance is a collagen-directed substance.

8. A method for producing cells having protein adhesion properties, comprising adsorbing the compound according to any one of claims 1 to 7 to the cells.

9. An agent for imparting protein adhesion properties to cells, comprising the compound according to any one of claims 1 to 7.

10. A kit for imparting protein adhesion properties to cells, comprising the compound according to any one of claims 1 to 7.

11. A cell adsorbed with the compound according to any one of claims 1 to 7.

12. A graft containing the cells according to claim 11.

13. The graft according to claim 12, which is in the form of cell mass, organoid, three-dimensional tissue, or paste.
